(19) Europäisches Patentamt
European Patent Office
Office européen des brevets

(11) Veröffentlichungsnummer: **0 053 358**
**B1**

(12) **EUROPÄISCHE PATENTSCHRIFT**

(45) Veröffentlichungstag der Patentschrift: **09.11.83**

(51) Int. Cl.³: **C 07 C 43/17**, C 07 C 41/06

(21) Anmeldenummer: **81109920.9**

(22) Anmeldetag: **26.11.81**

(54) **Verfahren zur Herstellung von 2-Alkenyl-1,1,2-trifluor-2-halogen-ethyl-ethern.**

(30) Priorität: **03.12.80 DE 3045473**

(43) Veröffentlichungstag der Anmeldung: **09.06.82 Patentblatt 82/23**

(45) Bekanntmachung des Hinweises auf die Patenterteilung: **09.11.83 Patentblatt 83/45**

(84) Benannte Vertragsstaaten: **DE FR GB IT**

(56) Entgegenhaltungen:
**DE - C - 852 541**
**US - A - 3 453 334**

**CHEMICAL ABSTRACTS, Vol. 53, 1959, Columbus, Ohio, USA A.D. PETROV et al. "Fluorosilicon organic compounds. Fluorosilicon organic ethers" column 1121c**

(73) Patentinhaber: **HOECHST AKTIENGESELLSCHAFT, Postfach 80 03 20, D-6230 Frankfurt am Main 80 (DE)**

(72) Erfinder: **von Werner, Konrad, Dr., Talhausen 11 1/3, D-8261 Burgkirchen/Alz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 053 358 B1

BUNDESDRUCKEREI BERLIN

# Verfahren zur Herstellung von 2-Alkenyl-1,1,2-trifluor-2-halogenethyl-ethern

Die Erfindung betrifft ein Verfahren zur Herstellung von 2-Alkenyl-1,1,2-trifluor-2-halogenethyl-ethern der Formel

$$R^1HC{=}CR^2{-}CHR^3{-}O{-}CF_2{-}CFXH,$$

worin $R^1$, $R^2$ und $R^3$ Wasserstoffatome oder Alkylgruppen mit 1 bis 3 C-Atomen bedeuten und X gleich Cl oder F ist, durch Umsetzung von Alkoholen der Formel

$$R^1HC{=}CR^2{-}CHR^3{-}OH$$

mit Fluorolefinen der Formel $CFX{=}CF_2$, worin $R^1$, $R^2$, $R^3$ und X die obengenannte Bedeutung besitzen, in Gegenwart eines Alkalimetallhydroxids als Katalysator.

Es ist bekannt, daß sich fluorhaltige Ether durch basenkatalysierte Addition von aliphatischen oder aromatischen Alkoholen an Tetrafluorethylen oder Chlortrifluorethylen gewinnen lassen. Es ist weiterhin bekannt, daß sich Allylalkohol in Gegenwart von KOH als Katalysator, welches als gesättigte Lösung im Alkohol vorliegt, mit Chlortrifluorethylen bei Normaldruck umsetzen läßt [J. Amer. Chem. Soc. 72 (1950), 4480 bis 4482], wobei zur Erzielung guter Ausbeuten jedoch sehr erhebliche Katalysatormengen eingesetzt werden müssen. Die entsprechende Etherbildung mit Tetrafluorethylen gelingt allerdings nur unter Anwendung von erhöhten Temperaturen und/oder Drücken [Doklady Akad. Nauk S.S.S.R 121 (1958), 307 bis 310; referiert in C. A. Vol. 53 (1959), 1121c]. Ein erheblicher Nachteil der genannten Verfahren zur Umsetzung von Allylalkohol besteht darin, daß durch die notwendige Verwendung großer Mengen des alkalischen Katalysators und/oder Anwendung erhöhter Temperaturen störende Nebenreaktionen auftreten, welche einen Verbrauch von Katalysator und Fluorolefin bewirken. Eine solche Nebenreaktion ist beispielsweise die Verseifung

$$CFX{=}CF_2 + 3\,KOH \rightarrow HFXC{-}CO_2K + 2\,KF + H_2O.$$

Es besteht daher die Aufgabe, das bekannte Verfahren der Umsetzung von Allylalkohol mit Chlortrifluorethylen oder Tetrafluorethylen so zu gestalten, daß die genannten Nachteile beseitigt werden.

Dies gelingt durch ein Verfahren der eingangs genannten Art, das dadurch gekennzeichnet ist, daß die Umsetzung in Gegenwart eines N,N-Dialkylcarbonsäureamids der Formel

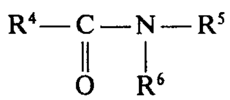

worin $R^4$ ein Wasserstoffatom oder einen Alkylrest mit 1 bis 3 C-Atomen, $R^5$ und $R^6$ Alkylreste mit 1 bis 3 C-Atomen bedeuten oder $R^4$ und $R^5$ zusammen eine cyclische Methylenbrücke $-(CH_2)_y-$, wobei y gleich 2 bis 4 ist, bilden,
als Lösungsmittel und unter intensiver Durchmischung der Reaktionspartner durchgeführt wird.

Die genannten Lösungsmittel aus der Gruppe der N,N-Dialkylcarbonsäureamide sind acyclische Verbindungen der Formel

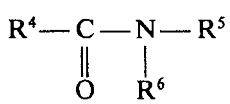

worin $R^4$ ein Wasserstoffatom oder ein Alkylrest mit 1 bis 3 C-Atomen ist sowie $R^5$ und $R^6$ Alkylreste mit 1 bis 3 C-Atomen sind,
oder cyclische Amide der Formel

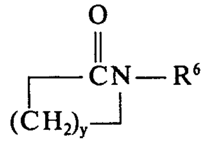

mit y=2 bis 4, vorzugsweise 3, wobei $R^6$ die vorgenannte Bedeutung hat. Bevorzugte Lösungsmittel sind N,N-Dimethylformamid, N,N-Dimethylacetamid und N-Methyl-2-pyrrolidon. Das Lösungsmittel soll möglichst wasserfrei sein. Um bei Normaldruck eine rasche Aufnahme des Fluorolefins zu

erreichen, ist im allgemeinen je Mol Alkohol eine Mindestmenge von 1 Mol Lösungsmittel im Falle des Chlortrifluorethylens und von 1,4 Mol Lösungsmittel im Falle des Tetrafluorethylens erforderlich. Diese Mengen können verringert werden, wenn unter Überdruck gearbeitet wird.

Um einen hohen Umsetzungsgrad des Alkohols zu erzielen, ist im allgemeinen die stöchiometrische Menge des Fluorolefins ausreichend. Zur quantitativen Umsetzung empfiehlt sich ein geringer Überschuß von maximal 5 Mol-% des Fluorolefins. Das Fluorolefin wird gasförmig eingeleitet. Bevorzugt wird das Verfahren mit Tetrafluorethylen durchgeführt.

Ein inniger Kontakt zwischen der Gasphase und der flüssigen Phase ist wesentlich für den Erfolg des Verfahrens. Eine intensive Durchmischung, bei der es darauf ankommt, möglichst kleine Gaspartikel mit großer Oberfläche zu schaffen und in der flüssigen Phase zu verteilen, läßt sich mit Hilfe effektiver Rühr- und Mischmethoden erreichen, beispielsweise durch hochtouriges Rühren und effektive Rührerkonstruktionen, effektives Schütteln, Einsatz von Hochfrequenz-Dispergiergeräten oder -mischgeräten, von Strahldüsenreaktoren oder durch Verwendung einer Füllkörper-Blasensäule nach dem Gegenstrom-Prinzip.

Die bei der Umsetzung angewandte Temperatur kann im Bereich von $-20$ bis $+60°C$, vorzugsweise von $+20$ bis $+40°C$ liegen. Die Reaktion zwischen Alkohol und dem Fluorolefin der Formel $CFX=CF_2$ verläuft unter Basenkatalyse in Gegenwart der genannten Carbonsäureamide als Lösungsmittel meist rasch und exotherm, so daß bei Normaldruck gearbeitet werden kann. Alkohole, welche geminal zur Hydroxygruppe durch Alkylgruppen substituiert sind ($R^3$=Alkyl), reagieren jedoch langsamer. In diesem Fall empfiehlt es sich, unter Druck zu arbeiten, wobei ein Druck von höchstens 3 bar ausreichend ist.

Als Alkalimetallhydroxid-Katalysatoren werden vorzugsweise Natrium- und Kaliumhydroxid eingesetzt. Die Katalysatormenge kann beim erfindungsgemäßen Verfahren deutlich verringert werden. Sie liegt beim Allylalkohol selbst im Bereich von 0,1 bis 10 Mol-%, vorzugsweise von 1 bis 5 Mol-%. Lediglich bei den alkylsubstituierten Derivaten des Allylalkohols, insbesondere bei solchen, die einen Alkylrest geminal zur OH-Gruppe tragen, ist ein etwas höherer Katalysatoreinsatz erforderlich, der jedoch höchstens 15 Mol-% beträgt. Die Alkalimetallhydroxide werden zweckmäßigerweise in feinverteilter Pulverform eingesetzt, können jedoch auch als handelsübliche Schuppen oder Pillen verwendet werden.

Vorzugsweise werden als Ausgangsalkohole diejenigen verwendet, bei denen in der obengenannten Formel die Reste $R^1$ oder $R^2$ oder $R^3$ eine Methylgruppe bedeuten. Besonders bevorzugt ist Allylalkohol selbst.

Infolge des erheblich geringeren Katalysatoreinsatzes werden beim erfindungsgemäßen Verfahren erheblich weniger Nebenprodukte gebildet, so daß eine erhöhte Ausbeute resultiert. Tetrafluorethylen kann nach dem erfindungsgemäßen Verfahren bei niedrigeren Drucken und Temperaturen umgesetzt werden als bisher nach dem Stand der Technik bekannt.

Die Produkte sind als Folge der hydrophoben fluorhaltigen Ethergruppe nicht wasserlöslich und lassen sich meist durch Zugabe von Wasser zum Reaktionsgemisch abtrennen. Eine besonders günstige Form der Isolierung ist häufig dadurch gegeben, daß sich die Siedepunkte vom Etherprodukt und Lösungsmittel so weit unterscheiden, daß eine einfache destillative Trennung möglich ist.

Die erfindungsgemäß zugänglichen Verbindungen besitzen eine reaktive C=C-Bindung und sind deshalb besonders als Zwischenprodukte zur Herstellung von Additionsprodukten mit guter Thermostabilität und hydrophoben Eigenschaften interessant.

So lassen sich in an sich bekannter Weise mit geeigneten Katalysatoren Alkohole und primäre oder sekundäre Amine addieren. Durch Hydrosilylierung, also Anlagerung von $R_3SiH$-Verbindungen an die Doppelbindung, können Silane, die die Oberflächenspannung des Wassers erniedrigen, hergestellt werden. Ferner gelingt die Einführung eines Perfluoralkylrestes unter Bildung von gesättigten Derivaten der Formel

$$R_f-(R^1)HC-CH(R^2)-CH(R^3)-O-CF_2-CFXH$$

durch Reaktion mit Perfluoralkylsulfonylchloriden $R_fSO_2Cl$, gefolgt von katalytischer Hydrierung.

Ein weiteres Anwendungsgebiet betrifft den Einsatz als Comonomere für fluorhaltige Copolymere.

Die Erfindung wird anhand der folgenden Beispiele demonstriert. Die Struktur der angegebenen Produkte wurde durch $^1H$- und $^{19}F$-Kernresonanzsprektren sowie Infrarotspektren geprüft.

## Beispiel 1

Ein zylindrischer Glasreaktor (Höhe 300 mm, Durchmesser 100 mm), der mit einem Labor-Hochfrequenzdispergator (Typ »Ultra-Turrax®«), einem Thermometer, einer Gasausgangsöffnung am Kopf sowie einer Gaseinleitung mit Glasfritte, welche sich direkt unter dem Kopf des Dispergators befindet, ausgestattet ist, wird mit 58 g (1,0 Mol) Allylalkohol, 100 ml wasserfreiem Dimethylformamid und 3,3 g KOH-Pulver (KOH-Gehalt 85%) (0,05 Mol) beschickt. Nun wird unter intensivem Durchmischen (Rotor-Umdrehungszahl 800 U/min) ein Tetrafluorethylen Strom so eingeleitet, daß ein am Gasauslaß

befindlicher Blasenzähler kein Abgas zeigt. Die Innentemperatur wird durch Kühlung des Reaktors mit einem Eisbad auf 30 bis 35°C gehalten. Die Gasaufnahme ist nach 40 min beendet, die Gewichtszunahme der Mischung beträgt 102 g. Das Reaktionsgemisch wird mit 1 l Wasser verdünnt und das abgeschiedene Rohprodukt zweimal mit je 500 ml $H_2O$ gewaschen. Nach Trocknen mit $CaCl_2$ wird bei Normaldruck destilliert. Man erhält 148,2 g $CH_2=CH-CH_2OCF_2CF_2H$ (Ausbeute 93,8% der Theorie) als farblose Flüssigkeit mit Kp. 76°C. Reinheit nach gaschromatographischer Analyse (GC): 99,6%.

## Beispiele 2 bis 8

Weitere Ergebnisse, die mit der in Beispiel 1 beschriebenen Verfahrensweise erhalten wurden, sind in der folgenden Tabelle zusammengefaßt. In Beispiel 8 wurde der Katalysator in kleinen Portionen im Laufe von 30 min zugegeben.

Tabelle

| Beispiel Nr. | Alkohol (je 1 Mol) | KOH (Mol-%) | DMF (ml) | Produkt | Ausbeute (%) | Kp. (° C) |
|---|---|---|---|---|---|---|
| 2 | $CH_2=CH-CH_2OH$ | 5 | 65 | $CH_2=CH-CH_2-OCF_2CF_2H$ | 62 | 76 |
| 3 | $CH_2=CH-CH_2OH$ | 5 | 85 | $CH_2=CH-CH_2-OCF_2CF_2H$ | 81 | |
| 4 | $CH_2=CH-CH_2OH$ | 5 | 200 | $CH_2=CH-CH_2-OCF_2CF_2H$ | 94,5 | |
| 5 | $CH_2=CH-CH_2OH$ | 2,5 | 200 | $CH_2-CH-CH_2-OCF_2CF_2H$ | 94 | |
| 6 | $CH_2-CH=CH-CH_2OH$ | 5 | 150 | $CH_3-CH=CH-CH_2-OCF_2CF_2H$ | 97 | 105,5 |
| 7 | $CH_2=C(CH_3)-CH_2OH$ | 10 | 150 | $CH_2=C(CH_3)-CH_2-OCF_2CF_2H$ | 78 | 91 |
| 8 | $CH_2=CH-CH(CH_3)OH$ | 15 | 250 | $CH_2=CH-CH(CH_3)-OCF_2CF_2H$ | 47 | 89 |

DMF = N,N-Dimethylformamid.

## Beispiel 9

Ein 100 l-Kessel wird mit $N_2$ gespült und dann mit 17,4 kg technisch reinem Allylalkohol (290 Mol), 30 kg N-Methylpyrrolidon und 1,0 kg KOH (85%ig) beschickt. Nun wird über ein Einleitungsrohr Tetrafluorethylen zudosiert, wobei der Rührer auf 800 U/min eingestellt wird und die Temperatur der Mischung durch Wasserkühlung auf 26 bis 30°C gehalten wird. Nach 6 Stunden beträgt die Tetrafluorethylen-Aufnahme 30,2 kg.

Der Kesselinhalt wird in eine Destillationsapparatur überführt und man destilliert zunächst bei Normaldruck, dann im Vakuum bis zu einer Übergangstemperatur von 132°C bei 90 Torr. Es werden ca. 50 kg Rohprodukt erhalten, welches noch N-Methylpyrrolidon enthält. Nach zweimaligem Waschen mit je 50 l Wasser wird der Ether über $CaCl_2$ getrocknet und destilliert.

Ausbeute: 44,8 kg $CH_2=CH-CH_2-OCF_2CF_2H$ (94,5%).

## Beispiel 10

64 g $Ch_2=CH-CH(CH_3)OH$, 250 g N,N-Dimethylacetamid und 7,0 g NaOH-Schuppen werden in einem Schüttelautoklaven bei 3 bar Tetrafluorethylen-Druck und 40°C während 2 Stunden umgesetzt.

Aufarbeitung analog Beispiel 1 ergibt 124,6 g $CH_2=CH-CH(CH_3)OCF_2CF_2H$ (Ausbeute 76%). Reinheit nach GC: 98,4%.

## Beispiel 11

203 g Allylalkohol, 370 g Dimethylformamid und 15 g KOH-Pulver werden wie in Beispiel 1 mit Chlortrifluorethylen umgesetzt. Nach 60 min ist die $ClFC=CF_2$-Aufnahme beendet. Aufarbeitung durch Auswaschen und Destillation ergibt 537 g $CH_2=CH-CH_2-OCF_2CFClH$ (88% Ausbeute). Kp. 109°C.

## Patentanspruch

Verfahren zur Herstellung von 2-Alkenyl-1,1,2-trifluor-2-halogenethyl-ethern der Formel

$$R^1HC=CR^2-CHR^3-O-CF_2-CFXH,$$

worin $R^1$, $R^2$ und $R^3$ Wasserstoffatome oder Alkylgruppen mit 1 bis 3 C-Atomen bedeuten und X gleich Cl oder F ist, durch Umsetzung von Alkoholen der Formel

$$R^1HC=CR^2-CHR^3-OH$$

mit Fluorolefinen der Formel $CFX=CF_2$, worin $R^1$, $R^2$, $R^3$ und X die obengenannte Bedeutung besitzen, in Gegenwart eines Alkalimetallhydroxids als Katalysator, dadurch gekennzeichnet, daß die Umsetzung in Gegenwart eines N,N-Dialkylcarbonsäureamids der Formel

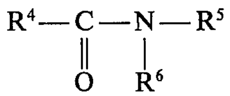

worin $R^4$ ein Wasserstoffatom oder einen Alkylrest mit 1 bis 3 C-Atomen, $R^5$ und $R^6$ Alkylreste mit 1 bis 3 C-Atomen bedeuten oder $R^4$ und $R^5$ zusammen eine cyclische Methylenbrücke $-(CH_2)_y-$,

wobei y = 2 bis 4 ist, bilden,
als Lösungsmittel und unter inniger Durchmischung der Reaktionspartner durchgeführt wird.

## Claim

A process for the preparation of a 2-alkenyl 1,1,2-trifluoro-2-halogenoethyl ether of the formula

$$R^1HC=CR^2-CHR^3-O-CF_2-CFXH$$

wherein $R^1$, $R^2$, and $R^3$ denote hydrogen atoms or alkyl groups having 1 to 3 C atoms and X is Cl or F, by reacting alcohols of the formula

$$R^1HC=CR^2-CHR^3-OH$$

with fluoroolefins of the formula $CFX=CF_2$ wherein $R^1$, $R^2$, $R^3$, and X are as defined above, in the presence of an alkali metal hydroxide as catalyst, which is characterised by carrying out the reaction in the presence of a N,N-Dialkylcarboxylic N-dialkylcarboxylic amide of the formula

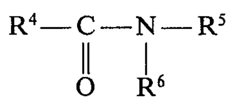

wherein $R^4$ denotes a hydrogen atom or an alkyl radical having 1 to 3 C atoms, $R^5$ and $R^6$ denote alkyl radicals having 1 to 3 C atoms or $R^4$ and $R^5$ together form a cyclic methylene bridge $-(CH_2)_y-$ in which y is 2 to 4, as the solvent, and with intimate mixing of the reactants.

## Revendication

Procédé pour préparer des oxydes d'alcène-2 yles et de trifluoro-1,1,2 halogéno-2 éthyles répondant à la formule:

$$R^1HC=CR^2-CHR^3-O-CF_2-CFXH$$

dans laquelle $R^1$, $R^2$ et $R^3$ représentent chacun un atome d'hydrogène ou un radical alkyle contenant de 1 à 3 atomes de carbone et X représente Cl ou F, par réaction d'alcools de formule

$$R^1HC=CR^2-CHR^3-OH$$

avec des fluoroléfines de formule $CFX=CF_2$, formules dans lesquelles $R^1$, $R^2$, $R^3$ et X ont les significations indiquées ci-dessus, en présence d'un hydroxyde de métal alcalin comme catalyseur,

procédé caractérisé en ce qu'on effectue la réaction en utilisant, comme solvant, un N,N-dialkylcarboxamide répondant à la formule

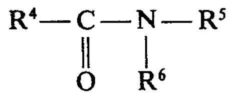

dans laquelle $R^4$ représente un atome d'hydrogène ou un radical alkyle contenant de 1 à 3 atomes de carbone, $R^5$ et $R^6$ représentent chacun un radical alkyle contenant de 1 à 3 atomes de carbone ou $R^4$ et $R^5$ forment ensemble un pont méthylénique cyclique $-(CH_2)_y-$ dans lequel y représente un nombre de 2 à 4,

et en mélangeant intimement les partenaires réactionnels.